# EUROPEAN PATENT APPLICATION

(11) **EP 4 400 068 A2**
(43) Date of publication of application: **17.07.2024**
(21) Application number: 23219147.8
(22) Date of filing: 21.12.2023
(51) Int. Cl.: A61B 34/10, A61B 34/20

(54) **PORT PLACEMENT RECOMMENDATION IN A SURGICAL ROBOTIC SYSTEM**

(30) Priority: 21.12.2022 US 202218086306
(71) Applicant: VERB SURGICAL INC., Santa Clara, CA 95054 (US)
(72) Inventor: SAXENA, Suryansh, Santa Clara, 95054 (US); SCHAEFFER, Shaun, Santa Clara, 95054 (US); CHUNDURU, Pranathi, Santa Clara, 95054 (US); SHINDE, Rohit, Irvine, 92618 (US); ISUKAPATI, Isaac, Redwood City, 94065 (US); JABLONOWSKI, Robert, Redwood City, 94065 (US); OU, Yusi, Redwood City, 94065 (US); NARASIMHAN, Narendran, Santa Clara, 95054 (US)
(74) Representative: Carpmaels & Ransford LLP

(57) **Abstract**

For a surgical robotic system, a machine-learned model is used to determine port placement. For example, a machine-learned model indicates internal regions based on input of external measurements on a patient. An overlap of an inverse-kinematics-based operation region of the surgical robotic system and the internal regions of the patient is optimized based on port placement. In an alternative or additional approach, information about the surgeon (e.g., handedness) is used with the machine-learned model to determine port placement.

## Description

### FIELD

Embodiments relate to recommendation for port placement in a surgical robotic system.

### BACKGROUND

Minimally invasive surgery (MIS), such as laparoscopic surgery, involves techniques intended to reduce tissue damage during a surgical procedure. MIS may be performed with robotic systems that include one or more robotic arms for manipulating surgical tools based on commands from a remote operator. MIS usage may be broadly divided into three parts: first, the surgeon creates a workspace in the abdominal cavity by lifting the abdominal wall via insufflation. Next, the surgeon makes small incisions known as ports at various locations in the abdominal wall and inserts the appropriate surgical instruments/tools through these ports. Finally, the surgeon performs the procedure inside the patient's body by maneuvering the surgical tools via surgical console.

Patient positioning and port placement play an instrumental role in safe and successful completion of these surgical procedures. Non-optimal port locations make it difficult to operate on the target anatomical structures and may require the placement of additional ports to successfully complete of the surgical procedure. Optimal port placement requires deeper understanding of the anatomical target locations, the functional purpose of each port, and the associated surgical tools in the overall surgical procedure. Often, surgeons make use of vendor developed port placement guidelines in conjunction with qualitative data on patient anatomy (e.g., measured distances from the landmarks) to determine the port placement. However, sole reliance on these techniques may result in port misplacement as the specific internal anatomical structures are not visible to the naked eye and target anatomical locations vary from patient to patient.

### SUMMARY

By way of introduction, the preferred embodiments described below include methods, systems, instructions, and computer readable media for port placement. A machine-learned model is used to determine the port placement. For example, a machine-learned model indicates internal regions based on input of external measurements on a patient. An overlap of an inverse-kinematics-based operation region of the surgical robotic system and the internal regions of the patient is optimized based on port placement. In an alternative or additional approach, information about the surgeon (e.g., handedness) is used with the machine-learned model to determine port placement.

In a first aspect, a method is provided for port placement for a surgical robotic system. A plurality of external patient measurements of a patient is input to a machine-learned model. In response to the inputting, the machine-learned model outputs one or more internal volumes of the patient. Overlap of an operating region of the surgical robotic system with the one or more internal volumes is optimized based on the port placement. An indication of the port placement from the optimized overlap is displayed.

In one embodiment, the external patient measurements are height, weight, gender, body mass index, abdominal width, and pelvic location. The output may be a centroid and radius representing the internal volume.

Various machine-learned models may be used. For example, a regression-trained model is used. As another example, a multi-layer perceptron neural network is used.

In an embodiment, the optimization includes identifying the operating region of the surgical robotic system with inverse kinematics. As another embodiment, the optimization includes identifying a part of the one or more internal volumes outside of the operating region for a port location for each of a plurality of arms of the surgical robotic system, identifying the arm of the surgical robotic system associated with a larger part, and changing the port location.

As one embodiment, the indication is displayed as part of a pre-operative plan.

According to a further embodiment, a profile of a surgeon is input to the machine-learned model. The one or more internal volumes are output in response to the inputting of the profile and the external patient measurements. The one or more internal volumes account for the profile of the surgeon.

In a second aspect, a method is provided for port placement for a surgical robotic system. A profile of a surgeon is input to a machine-learned model. In response to the inputting, the machine-learned model outputs one or more internal volumes of a patient. The one or more internal volumes account for the profile of the surgeon. An indication of port placement based on the one or more internal volumes is displayed.

In one embodiment, the internal volumes are regions for surgical interaction based on a characteristic of the surgeon reflected in the profile. Example characteristics include handedness of the surgeon or surgical trajectory used by the surgeon.

According to another embodiment, external measurements of the patient are also input to the machine-learned model, which outputs the one or more internal volumes based on the external measurements of the patient.

In yet another embodiment, overlap of an operating region of the surgical robotic system with the one or more internal volumes is optimized based on the port placement. The indication of the port placement from the optimized overlap is displayed.

In a third aspect, a surgical robotic system is provided for port placement. A robotic arm is configured to hold and operate a surgical tool. A processor is configured to determine a location of a port for the surgical tool to enter a patient. The location is determined from an output of artificial intelligence generated in response to input of patient measurements and surgeon information.

As one embodiment, the output of the artificial intelligence is one or more internal regions of the patient. The one or more internal regions are regions for interaction of the surgical tool with the patient. The processor is configured to optimize the location of the port based on overlap of the one or more internal regions and an operating region accessible by the surgical tool using the robotic arm. The operating region is based on inverse kinematics.

In another embodiment, the surgeon information includes a handedness of a surgeon controlling the robotic arm, and the one or more internal regions being positioned based on the handedness. As another embodiment, the surgeon information includes a trajectory of a surgeon controlling the robotic arm. The trajectory is a sequence or route to be used by the surgeon during operation on the patient with the surgical tool, and the one or more internal regions being positioned based on the trajectory.

In yet another embodiment, the patient measurements are external measurements of the patient.

As another embodiment, the artificial intelligence is a machine-learned regression model or a machine-learned neural network.

The present invention is defined by the following claims, and nothing in this section should be taken as a limitation on those claims. Further aspects and advantages of the invention are discussed below in conjunction with the preferred embodiments and may be later claimed independently or in combination.

### BRIEF DESCRIPTION OF THE DRAWINGS

The embodiments of the invention are illustrated by way of example and not by way of limitation in the figures of the accompanying drawings in which like references indicate similar elements. It should be noted that references to "an" or "one" embodiment of the invention in this disclosure are not necessarily to the same embodiment, and they mean at least one. Also, in the interest of conciseness and reducing the total number of figures, a given figure may be used to illustrate the features of more than one embodiment of the invention, and not all elements in the figure may be required for a given embodiment.
Figure 1 illustrates an embodiment of a cart-based robotic system arranged for diagnostic and/or therapeutic use;
Figure 2 illustrates an embodiment of a table-based robotic system configured for a procedure;
Figure 3 is a block diagram of another embodiment of a surgical robotic system using a machine-learned model to determine port placement;
Figure 4 is a flow chart diagram of one embodiment of a method for determining port placement with artificial intelligence;
Figure 5 illustrates example port placement options and corresponding overlaps of robotic operating region and internal regions of the patient on which the surgery is to be performed;
Figure 6 illustrates example division of a port placement process into acquiring training data as data preparation, training of the artificial intelligence model, and pre-operational planning for port placement using the trained artificial intelligence model;
Figure 7 illustrates example data preparation for identifying internal regions as workspaces for a surgery;
Figure 8 illustrates an example internal region as a workspace;
Figure 9 illustrates multiple workspaces identified based on clustering according to one embodiment;
Figure 10 illustrates example workspaces and their corresponding part of the surgical procedure for gastric bypass surgery using a robotic surgical system;
Figure 11 illustrates spatial transform for normalization of data used to generate training data according to an embodiment;
Figures 12 and 13 illustrate example use of a convolutional neural network for generation of training data;
Figure 14 illustrates an example division of the determination of port placement into acquiring external measurements of the patient, predicting internal regions, and analysis of workspace of the surgical robotic system relative to the internal regions for determining port placement;
Figure 15 illustrates one embodiment of training of a regression model as the artificial intelligence;
Figure 16 illustrates an embodiment of a neural network as the artificial intelligence;
Figure 17 illustrates optimization based on port placement according to one embodiment;
Figure 18 illustrates an example amount overlap given a port position for optimization;
Figure 19 illustrates one embodiment of optimization based on port placement; and
Figure 20 illustrates an example of overlap of a workspace of a robotic surgical system with internal volumes of the patient for surgical operation after optimization of the port placement.

### DETAILED DESCRIPTION

A pre-surgical assistance system framework is provided for optimal port placement planning. A machine learning model in the framework makes use of labeled telemetry data and clinical (e.g., age, height, abdominal width) and surface measurements for training to accurately identify anatomical workspace volumes or to directly indicate port location. For a given surgery, the telemetry data for any minimally invasive surgical platform is used to annotate and/or label regions of interest in the telemetry data, identify anatomical workspace volumes through unsupervised segmentation, use 3-dimensional surface reconstruction algorithms to fit a representative geometrical shape for each identified anatomical workspace, create machine learning models that best explain the functional relationship between the external anatomical measurements and geometrical properties (centroid and radius) of anatomical workspace volumes, and evaluate the efficacy of predicted workspaces using inverse robot kinematics.

The example surgical procedure used herein is Roux-en-Y Gastric Bypass (RYGB). The port placement may be used for other bypass surgeries, such as gastrectomy or gastric bypass. The port placement may be used for any other robotic surgical procedures, such as Cholecystectomy.

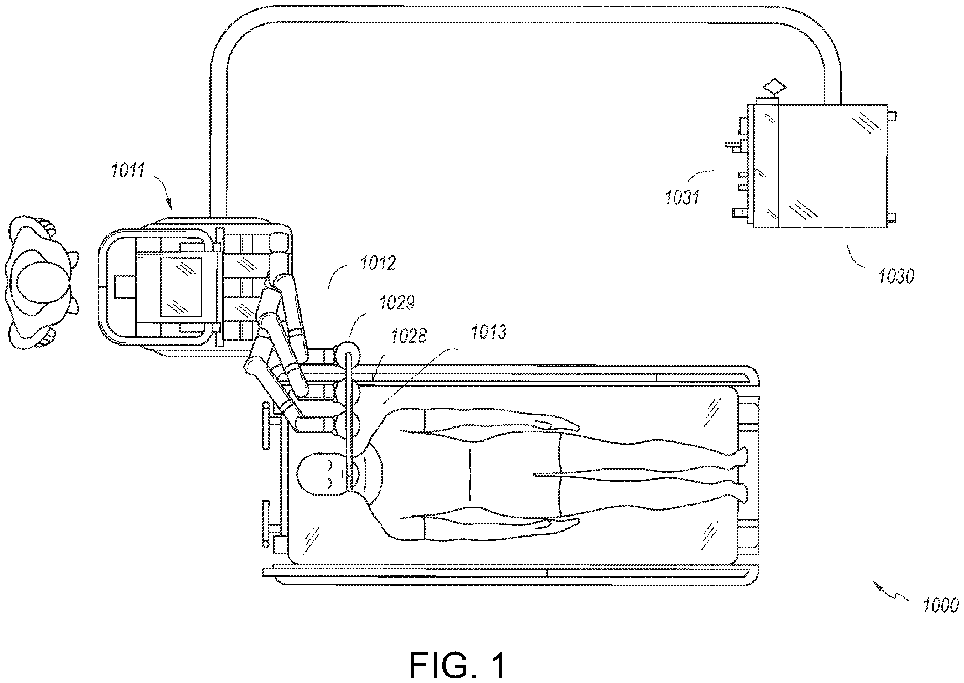

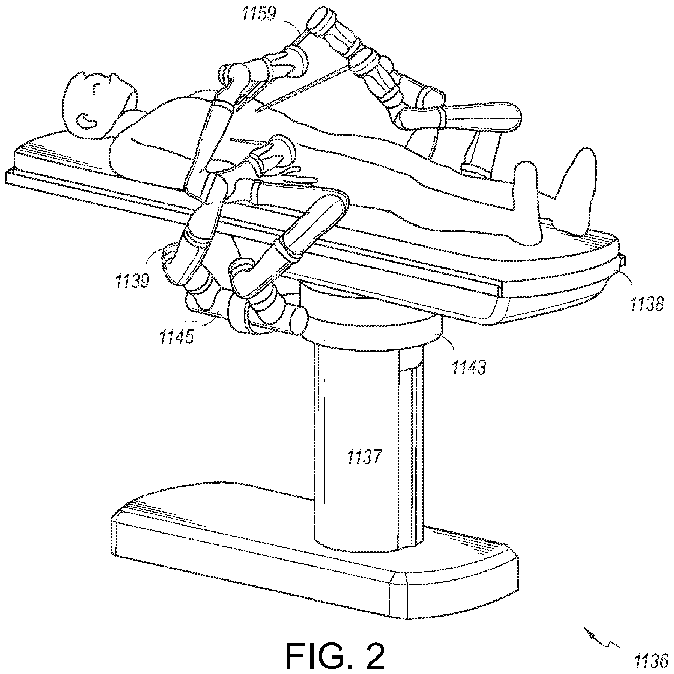

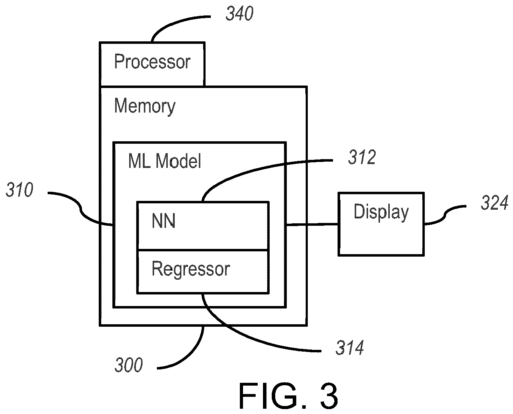

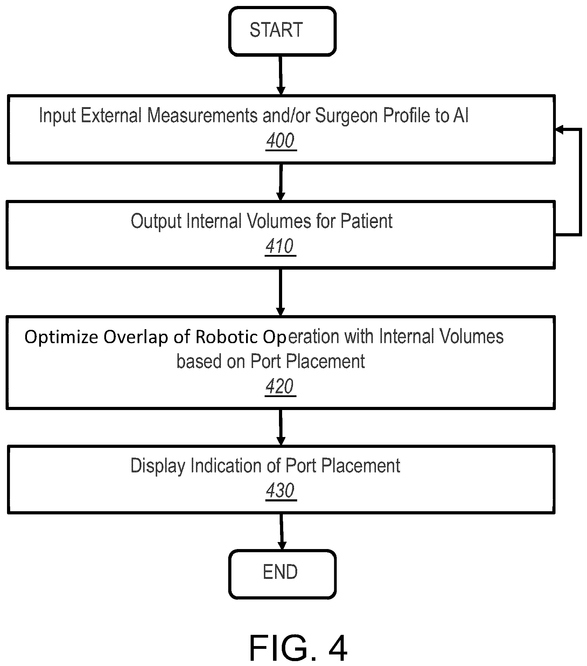

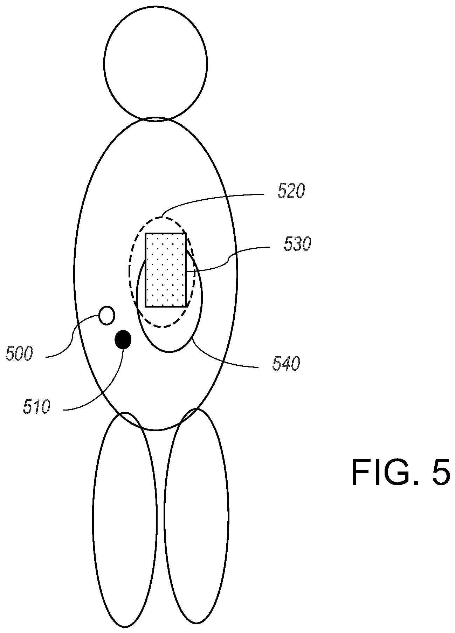

The discussion below first introduces an example robotic surgery system (see Figures 1 and 2). Figures 3 and 4 are directed to use of a machine-learned model in port placement determination. Figures 5-20 show embodiments for training the machine-learned model, the machine-learned model, and optimization of port placement based on the trained machine-learned model. Figures 5-20 use an example where the machine-learned model outputs internal regions for surgery. In other examples, the machine-learned model directly outputs the port placement.

The robotic surgical system may have any arrangement, such as one or more robotic arms. One or more surgical instruments may be used, such as graspers, clamps, endoscope, and/or scalpel instruments. Figures 1 and 2 show an example robotic surgery system. Other surgical instruments, robotic arms, and/or robotic surgery systems may be used.

Figure 1 shows an embodiment of a surgical robotic system. The robotically-enabled medical system may be configured in a variety of ways depending on the particular procedure. In Figure 1, the surgical robotic system is a cart-based robotically-enabled system 1000 arranged for a diagnostic and/or therapeutic surgery, such as bronchoscopy. The system 1000 may include a cart 1011 having one or more robotic arms 1012 to deliver a medical instrument, such as a steerable endoscope 1013 and/or therapeutic tools. The cart 1011 may be positioned proximate to the patient's upper torso to provide access to an access point. Similarly, the robotic arms 1012 may be actuated to position the instruments. The arrangement in Figure 1 with the arms 1012 repositioned may be utilized when performing a gastro-intestinal (GI) procedure with a gastroscope, RYGB, or a specialized endoscope for GI procedures.

Once the cart 1011 is properly positioned, the robotic arms 1012 may insert the steerable endoscope 1013 into the patient robotically, manually, or a combination thereof. The steerable endoscope 1013 may include at least two telescoping parts, such as an inner leader portion and an outer sheath portion, each portion coupled to a separate instrument driver from the set of instrument drivers 1028, each instrument driver coupled to the distal end of an individual robotic arm 1012. This linear arrangement of the instrument drivers 1028, which facilitates coaxially aligning the leader portion with the sheath portion, creates a "virtual rail" 1029 that may be repositioned in space by manipulating the one or more robotic arms 1012 into different angles and/or positions. The virtual rails described herein are not any physical structure of the system but an arrangement of other structures. Translation of the instrument drivers 1028 along the virtual rail 1029 telescopes the inner leader portion relative to the outer sheath portion or advances or retracts the endoscope 1013 from the patient. The angle of the virtual rail 1029 may be adjusted, translated, and pivoted based on clinical application or physician preference. For example, in bronchoscopy, the angle and position of the virtual rail 1029 as shown represents a compromise between providing physician access to the endoscope 1013 while minimizing friction that results from bending the endoscope 1013 into the patient's mouth. Similarly, for RYGB, the endoscope is inserted through a port in the patient, so the angle and position of the virtual rail 1029 is oriented about that access point. The virtual rail may not be used for some procedures, such as RYGB.

The endoscope 1013 may be directed within the patient after insertion using precise commands from the robotic system until reaching the target destination or operative site. To enhance navigation and/or reach the desired target, the endoscope 1013 may be manipulated to telescopically extend the inner leader portion from the outer sheath portion to obtain enhanced articulation and greater bend radius. The use of separate instrument drivers 1028 also allows the leader portion and sheath portion to be driven independently of each other.

The system 1000 may also include a movable tower 1030, which may be connected via support cables to the cart 1011 to provide support for controls, electronics, fluidics, optics, sensors, and/or power to the cart 1011. Placing such functionality in the tower 1030 allows for a smaller form factor cart 1011 that may be more easily adjusted and/or re-positioned by an operating physician and his/her staff. Additionally, the division of functionality between the cart/table and the support tower 1030 reduces operating room clutter and facilitates improving clinical workflow. While the cart 11 may be positioned close to the patient, the tower 1030 may be stowed in a remote location to stay out of the way during a procedure.

In support of the robotic systems described above, the tower 1030 may include component(s) of a computer-based control system that stores computer program instructions, for example, within a non-transitory computer-readable storage medium such as a persistent magnetic storage drive, solid state drive, etc. The execution of those instructions, whether the execution occurs in the tower 1030 or the cart 1011, may control the entire system or sub-system(s) thereof. For example, when executed by a processor of the computer system, the instructions may cause the components of the robotics system to actuate the relevant carriages and arm mounts, actuate the robotics arms, and control the medical instruments. For example, in response to receiving the control signal, the motors in the joints of the robotics arms may position the arms into a certain posture.

The tower 1030 may also include a pump, flow meter, valve control, and/or fluid access to provide controlled irrigation and aspiration capabilities to the system that may be deployed through the endoscope 1013. The tower 30 may include a voltage and surge protector designed to provide filtered and protected electrical power to the cart 11, thereby avoiding placement of a power transformer and other auxiliary power components in the cart 1011, resulting in a smaller, more moveable cart 1011. The tower 1030 may also include support equipment for the sensors deployed throughout the robotic system 1000. Similarly, the tower 1030 may also include an electronic subsystem for receiving and processing signals received from deployed electromagnetic (EM) sensors. The tower 1030 may also be used to house and position an EM field generator for detection by EM sensors in or on the medical instrument.

The tower 1030 may also include a console 1031 in addition to other consoles available in the rest of the system, e.g., console mounted on top of the cart. The console 1031 may include a user interface and a display screen, such as a touchscreen, for the physician operator. Consoles in the system 1000 are generally designed to provide both robotic controls as well as preoperative and real-time information of the procedure, such as navigational and localization information of the endoscope 13. When the console 1031 is not the only console available to the physician, it may be used by a second operator, such as a nurse, to monitor the health or vitals of the patient and the operation of the system 1000, as well as to provide procedure-specific data, such as navigational and localization information. In other embodiments, the console 1031 is housed in a body that is separate from the tower 1030.

Embodiments of the robotically-enabled medical system may also incorporate the patient's table. Incorporation of the table reduces the amount of capital equipment within the operating room by removing the cart, which allows greater access to the patient. Figure 2 illustrates an embodiment of such a robotically-enabled system. The system 1136 includes a support structure or column 1137 for supporting platform 1138 (shown as a "table" or "bed") over the floor. Much like in the cart-based systems, the end effectors of the robotic arms 1139 of the system 1136 comprise instrument drivers that are designed to manipulate an elongated medical instrument, such as an endoscope, grasper, and/or scalpel.

The column 1137 may include one or more carriages 1143 shown as ring-shaped in the system 1136, from which the one or more robotic arms 1139 may be based. The carriages 1143 may translate along a vertical column interface that runs the length of the column 1137 to provide different vantage points from which the robotic arms 1139 may be positioned to reach the patient. The carriage(s) 1143 may rotate around the column 1137 using a mechanical motor positioned within the column 1137 to allow the robotic arms 1139 to have access to multiples sides of the table 1138, such as, for example, both sides of the patient. In embodiments with multiple carriages 1143, the carriages 1143 may be individually positioned on the column 1137 and may translate and/or rotate independently of the other carriages. While the carriages 1143 need not surround the column 1137 or even be circular, the ring-shape as shown facilitates rotation of the carriages 1143 around the column 1137 while maintaining structural balance. Rotation and translation of the carriages 1143 allows the system 1136 to align the medical instruments into different access points on the patient. In other embodiments (not shown), the system 1136 can include a patient table or bed with adjustable arm supports in the form of bars or rails extending alongside it. One or more robotic arms 1139 (e.g., via a shoulder with an elbow joint) can be attached to the adjustable arm supports, which can be vertically adjusted. By providing vertical adjustment, the robotic arms 1139 are advantageously capable of being stowed compactly beneath the patient table or bed, and subsequently raised during a procedure.

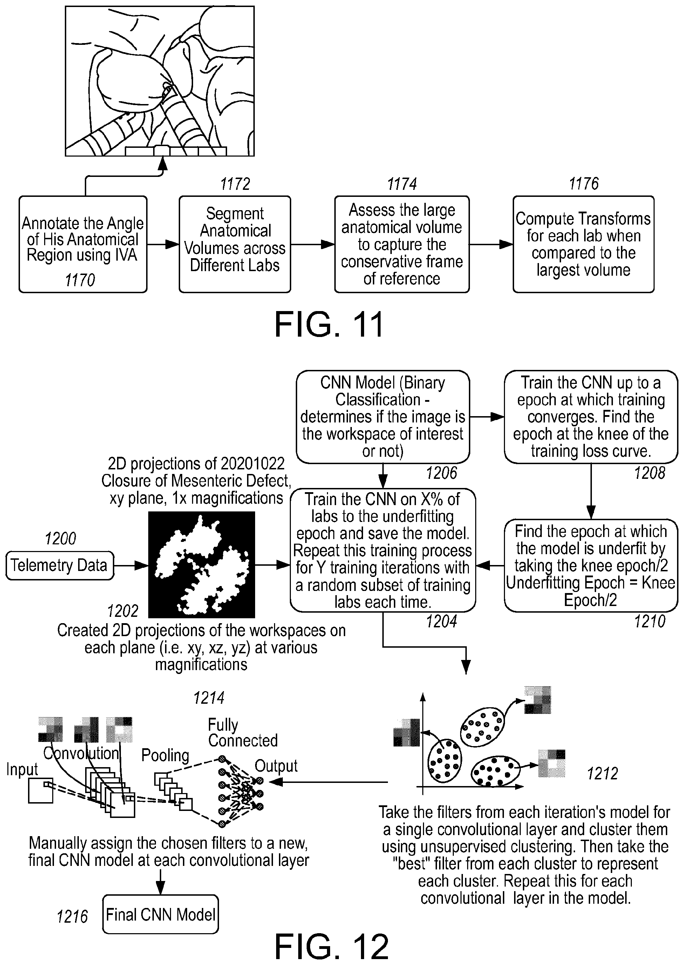

The robotic arms 1139 may be mounted on the carriages 1143 through a set of arm mounts 1145 including a series of joints that may individually rotate and/or telescopically extend to provide additional configurability to the robotic arms 1139. Additionally, the arm mounts 1145 may be positioned on the carriages 1143 such that, when the carriages 1143 are appropriately rotated, the arm mounts 1145 may be positioned on either the same side of the table 1138, on opposite sides of the table 1138 (as shown in Figure 11), or on adjacent sides of the table 1138 (not shown).

The column 1137 structurally provides support for the table 1138, and a path for vertical translation of the carriages 1143. Internally, the column 1137 may be equipped with lead screws for guiding vertical translation of the carriages, and motors to mechanize the translation of the carriages 1143 based on the lead screws. The column 1137 may also convey power and control signals to the carriages 1143 and the robotic arms 1139 mounted thereon.

In one embodiment, the robotic surgical system of Figure 2 is used for RYGB. In a laparoscopic procedure, through small incision(s) in the patient's abdominal wall, minimally invasive instruments may be inserted into the patient's anatomy. In some embodiments, the minimally invasive instruments include an elongated rigid member, such as a shaft, which is used to access anatomy within the patient. After inflation of the patient's abdominal cavity, the instruments may be directed to perform surgical or medical tasks, such as grasping, cutting, ablating, suturing, etc. In some embodiments, the instruments include a scope, such as a laparoscope for a viewing task, not necessarily surgical in the sense of operating directly on tissue, one or more graspers for manipulating intestine, and a scalpel for creating a bypass. Figure 2 illustrates an embodiment of a robotically-enabled table-based system configured for a laparoscopic procedure, such as RYGB. As shown in Figure 2, the carriages 1143 of the system 1136 may be rotated and vertically adjusted to position pairs of the robotic arms 1139 on opposite sides of the table 1138, such that instruments 1159 may be positioned using the arm mounts 1145 to be passed through minimal incisions (ports) on both sides of the patient to reach his/her abdominal cavity. RYGB may then be performed using one or more drivers with the surgical instruments or tools.

Figure 3 shows one embodiment of a surgical robotic system for port location determination. The system is a workstation, computer, controller, server, tablet, or mobile device. In one embodiment, the system is part of the surgical robot, such as being in the tower 1030 or cart 1011. In another embodiment, the system communicates wirelessly and/or wired (e.g., over a computer network) with the robotic system 1000, 1136.

Via communications and/or physical connection, the system of Figure 3 connects with one or more of the robotic arms, such as connecting with the surgical robot or system 1000, 1136. Interaction with the arms 1139 of Figure 2 will be used in examples below, but interaction with other robotic arms (e.g., arms 1012 of Figure 1) may be used in other examples. The robotic arm(s) 1139 are configured to hold and operate surgical tool(s) 1159.

The surgical robotic system includes a processor 340, a memory 300, and display 342. Additional, different, or fewer devices may be provided.

The processor 340 is a general processor, application specific integrated circuit, field programmable gate array, digital signal processor, controller, artificial intelligence processor, tensor processor, graphics processing unit, digital circuit, analog circuit, combinations thereof, and/or other now known or later developed processor for determining a port location using artificial intelligence. The processor 340 is configured by software, hardware, and/or firmware to apply the artificial intelligence and/or determine the port location for each of one or more robotic arms 1139.

The processor 340 is configured to determine the location of one or more ports for a surgery as part of planning. Since different patients have different sizes and/or arrangements of internal organs, different patients may have different locations for a more or most optimal location of each port. Since different surgeons perform a same surgery (e.g., RYGB) differently due to handedness (e.g., left or right-handed) and/or trajectory (e.g., different sequence of events, route to locations within the body, and/or different interactions with tissue), different surgeons may have different locations for a more or most optimal location of each port. The processor 340 is configured to determine a location of a port for the surgical tool to enter a patient for each of the robotic arms 1139 to be used in a surgery.

The processor 340 is configured to determine the port location using an artificial intelligence. In one embodiment, the artificial intelligence outputs the port location in response to input of information. In another embodiment, the artificial intelligence outputs information used to then determine an optimal port location, such as outputting a region reachable by the robotic arm 1139 or arms 1139 and/or outputting one or more internal regions of the patient at which the instrument 1159 interacts with tissue of the patient during surgery. The processor 340 is configured to apply the artificial intelligence and use the output to then determine the port location. The processor 340 may be configured to perform other processing, such as pre-processing (e.g., normalization or scaling) of the input to the artificial intelligence or post-processing (e.g., applying heuristics and/or a procedure to sequentially determine a combination of port locations for different robotic arms 1139) of the output of the artificial intelligence.

The artificial intelligence outputs in response to an input. Any of various inputs may be used, such as patient measurements with or without other patient clinical data. The measurements may be from a pre-operative scan so may represent internal information. In one embodiment, the measurements are external to the patient. These external measurements are input without needing internal information for the patient. One or more of various external measurements may be used. For example, sixteen or more of rib length and width MCL and axillary insufflated, lateral workspace projected insufflated, lateral workspace arc insufflated, length of chest to pubis insufflated, lateral chest projected insufflated, chest height insufflated, chest circumference insufflated, circumference xiphoid insufflated, circumference mid (umbilicus) insufflated, circumference lower (hips) insufflated, pelvis height, gender, age, height, weight, body mass index, volume, abdominal width projected, abdominal width arc, abdominal height lower, abdominal height mid projected, abdominal height upper projected, abdominal length projected, abdominal length arc, lower abdominal length projected, lower abdominal length arc, ribs length MCL, ribs width MCL, ribs length axillary, ribs width axillary, latera workspace projected, lateral workspace arc, pelvis width (Iliac Crest) projected, pelvis width ASIS projected, circumference chest, circumference Xiphoid, circumference mid (umbilicus), circumference lower (hips), pelvis pubis length, height of pubis, pelvis height ASIS, abdominal wall thickness, abdominal width projected insufflated, abdominal width arc insufflated, abdominal height lower projected insufflated, abdominal height mid projected insufflated, abdominal height upper projected insufflated, abdominal height upper projected insufflated, and abdominal length projected insufflated are used. Other measures, whether actual measures or estimated measures, of the exterior of the patient may be used. In one embodiment, the measures input are at least height, weight, gender, body mass index, abdominal width, and pelvic location. Any number of and any specific external measurements may be used. Other information may be input, such as clinical data for the patient (family history, diagnosis, .. .) and/or personal health information (e.g., CT-scan data).

The input may alternatively or additionally be surgeon information. Handedness and/or trajectory for a given surgeon are input.

The artificial intelligence generates one or more outputs. For example, the output is the port location given in any coordinate system, such as relative to one or more landmarks on an exterior of the patient. As another example, the output is a list of locations of tissue interactions. In another example, the output is one or more regions for surgical interaction. The regions may be parameterized in any way, such as central location and radius, parameters defining a box, parameters defining a sphere, parameters defining an ovoid, or a mesh defining any shape of region where interaction occurs. As another example, the output is a region reachable by the surgical instrument from a given port location. The region may be parameterized in any way. In yet another example, a combination of regions reachable by all of the surgical instruments 1159 held by the robotic arms 1139 is output. As another example, a region of overlap or non-overlap between the locations of tissue interaction and robotic workspace is output.

The artificial intelligence is a machine-learned model 310. In the example embodiment of Figure 3, the machine-learned model 310 stored in the memory 300 is configured by training to receive the input (e.g., external measurements and/or surgeon information) and generate the output.

The machine-learned model 310 was previously trained using machine learning. Machine learning is an offline training phase where the goal is to identify an optimal set of values of learnable parameters of the model 310 that can be applied to many different inputs (i.e., previously unseen inputs for a given patient). These machine-learned parameters can subsequently be used during operation (testing or application phase) to rapidly generate the output. Once learned, the machine-learned model 310 is used in an online processing phase in which the inputs (e.g., external measurements and/or surgeon profile or information) are input, and the port location or information used to determine the port location is output based on the model values learned during the training phase.

Various types of machine learning models may be used, such as support vector machines, neural networks 312, Bayesian, regression 314, or another. In one embodiment, the model is a neural network, such as a fully connected neural network or a convolutional neural network. For example, a multi-layer perceptron neural network 312 is used. As another example, a regression model 314, such as linear, logistic, ridge, Lasso, polynomial, or Bayesian linear regression model is used. Any architecture or layer structure for machine learning may be used, such as U-Net, encoder-decoder, or image-to-image network. The architecture defines the structure, learnable parameters, and relationships between parameters. Different machine-learned models 310 may be used for different outputs, such as different models or a multi-task model to generate different internal regions.

The model 310 is machine trained. Deep or other machine training is performed. Many (e.g., hundreds or thousands) samples of inputs to the model and the ground truth outputs are collected as training data. For example, data from testing (e.g., on cadavers) or from usage (e.g., from surgeries performed on patients) is collected as training data. The inputs are measured, and the resulting output is measured during the testing or surgery. Simulation may be used to generate the training data. Experts may curate the training data, such as assigning ground truth for collected samples of inputs. Training data may be created from a guideline or statistical shape model. Many samples for a given surgery may be collected.

The machine training optimizes the values of the learnable parameters. Backpropagation, RMSprop, ADAM, or another optimization is used in learning the values of the learnable parameters of the model 310. Where the training is supervised, the differences (e.g., L1, L2, or mean square error) between the estimated output (e.g., centroid location and radius) and the ground truth output are minimized.

Once trained, the model 310 is applied before teleoperation for a given patient. For example, the machine-learned model 310 is used to determine the port location or locations, which location or locations are then used for surgery for a given patient by a given surgeon. The machine-learned model 310 is previously trained, and then used as trained. Fixed values of learned parameters are used for application. The learned values and network architecture determine the output from the input. During application for pre-operative planning for a patient, the same learned weights or values are used. The model and values for the learnable parameters are not changed from one patient to the next, at least over a given time (e.g., weeks, months, or years) or given number of surgeries (e.g., tens or hundreds). These fixed values and corresponding fixed model are applied sequentially and/or by different processors to inputs for different patients. The model may be updated, such as retrained, or replaced, but does not learn new values as part of application for a given patient.

In one embodiment, the output of the artificial intelligence is one or more internal regions of the patient (see Figures 5-20). The internal regions are regions for interaction of the surgical tool with the patient, so are anatomy based workspace rather than the workspace reachable by the surgical instrument 1159. The processor is configured to optimize the location of the port based on overlap of the one or more internal regions and an operating region accessible by the surgical tool 1159 using the robotic arm(s) 1012. The operating region or workspace of the tool 1159 is determined for the port location based on inverse kinematics. Inverse kinematics may be customized for any robotics architecture, making the framework robot hardware agnostic.

The output internal regions may be based on the various inputs. For example, different internal regions are generated for different surgeon characteristics, such as handedness and/or trajectory used. The trajectory is a sequence or route to be used by the surgeon during operation on the patient with the surgical tool 1159. The one or more internal regions are positioned based on the trajectory as tissue interaction from a particular direction may shift tissue. As another example, the different internal regions are generated for different patient characteristics. Different external measurements for the patient may lead to different internal regions (e.g., locations of the internal regions) for the patient in the surgery. The one or more internal regions are based on the patient characteristics. A combination of surgeon characteristic and patient characteristics may be input.

The processor 340 is configured to determine the port locations. For example, the amount of overlap of the workspace (operating region of the surgical tools 1159) and of the internal regions of the patient is minimized by adjusting locations of the ports. Each port is adjusted independently or the various ports are adjusted sequentially or in relation to each other.

Figure 4 shows one embodiment of a method for determining port locations for one or more robotic arms 1139 and corresponding surgical instruments 1159 for a given patient. A machine-learned model 310, as trained, is used to output information (e.g., internal regions (i.e., locations and size)) used to determine the port locations. In alternative embodiments, the machine-learned model 310 directly outputs the port locations in response to the input.

Patient positioning and port placement play an instrumental role in safe and successful completion of surgical procedures. For example, Figure 5 shows two different port locations 500, 510 possible for a surgical instrument 1139 where a dashed oval 520 represents a region reachable for one port location 500 and a solid line oval 540 represents a region reachable for another port location 510. The unique locations of various anatomical targets 530 along with the limited space for robot mobility requires careful pre-operative planning for optimal port placement, such as selecting the port location 500 for greater coverage of the targets 530. Optimal port placement uses the anatomical target locations, the functional purpose of each port, and the associated surgical tools in the overall surgical procedure. Each tool and corresponding robotic arm define a workspace based on inverse kinematics. The goal is to determine the port location providing the greatest or sufficient overlap of the workspace and the internal volumes of the patient on which the work is performed.

The method of Figure 4 is performed by the surgical robotic system of Figures 1, 2, or 3 or another surgical robotic system. In one embodiment, the surgical robotic system of Figure 3 performs the method. An interface receives the measurements or surgeon profile, such as from user input, transfer over a computer network, or loading from memory. A programmed processor 340 (also referred to here as processing logic or circuit) performs the input to the artificial intelligence in act 400 and generates the output in act 410 using the artificial intelligence. The processor optimizes overlap in act 420 and generates the indication for display or use in surgery. A display screen displays the indication in act 430. A non-transitory memory 300 may store instructions for the programmed processor 340 to determine the port location(s) and/or the machine-learned model or models 310.

The acts are performed in the order shown or a different order. Additional, different, or fewer acts may be provided. For example, act 420 is performed as part of acts 400 and 410 where robotic positioning information is input and the machine-learned model 310 outputs the port location directly. As another example, the processor 340 detects the type of surgical instrument 1159 connected to the robotic arm 1139 and selects the machine-learned model 310 to use for port location determination. In yet another example, acts for connecting the surgical instrument 1159 to the robotic arm 1139 and using the surgical robot at the determine port locations are provided.

In act 400, information is input to the machine-learned model. Various information may be gathered, such as patient measurements (e.g., external measurements). For example, the external patient measurements of height, weight, gender, body mass index, abdominal width, and pelvic location are input. Other information may include a profile of a surgeon. Information representing handedness, planned or used trajectory, or other characteristic of the surgeon in the surgery is input.

In act 410, the machine-learned model outputs one or more internal volumes of the patient. Multiple models may output a corresponding multiple different internal volumes, such as different models having been trained to output volumes for different tissue interactions or parts of the surgery. A sequence or hierarchy of models may be used, such as subsequent models using the internal volume or volumes of earlier models in the sequence or hierarchy as inputs to then output another internal volume. In other embodiments, one model outputs multiple (e.g., all) of the internal volumes. In response to input of the information for a given surgery (e.g., patient measurements and/or surgeon profile), the machine-learned model or models output one or more internal volumes of the patient.

The internal volumes are for a given surgical instrument 1159 or for multiple (e.g., all) of the instruments 1159 to be used in a surgery. The internal volumes designate regions where the surgical instrument(s) 1159 are to interact with tissue of the patient during the surgery.

In one example, the input is external measurements of the patient, such as sixteen or more different external measurements as actual direct measurements or predictions. A regression trained, multi-layer perceptron neural network, or other machine-learned model outputs multiple internal regions or a contiguous region of any shape (e.g., outputs a mesh) in response to input of the external measurements of the patient. In one embodiment, the output is a centroid and radius for one or more internal regions. Some internal regions may overlap. In other embodiments, a different (e.g., more complex) representation is output, such as an orientation and camera view points.

In another example, the input includes a profile of the surgeon. The handedness, trajectory used, or other information or characteristic of the surgeon in the given type of surgery is input. The machine-learned model, having been trained with training data including profile information, outputs internal volumes in response to the profile, such as in response to input of the profile and external measurements of the patient. The output internal volumes account for the profile of the surgeon. Some locations of tissue interaction may be different due to the profile. For example, different robotic arms 1139 are likely to hold different tools depending on handedness of the surgeon. The result may be a shift in locations of tissue interaction. The internal volumes are output as regions for surgical interaction based on a characteristic of the surgeon reflected in the profile.

In act 420, a processor optimizes overlap of an operating region of the surgical robotic system with the internal volumes. The placement of a port and design of the robotic arm 1139 and surgical instrument 1159 defines a region within the patient reachable by the surgical instrument 1159. This workspace may have blockages due to bone or avoidance of other tissue as well. The instrument workspace is a region based, in part, on the port placement identifiable with inverse kinematics. Different port locations may result in different size, shape, and/or position of the workspace for each surgical instrument to be used. Inverse kinematics is used to determine the workspace for each surgical instrument and corresponding possible port location.

The processor determines the overlap of the workspaces of the needed surgical instruments with the internal volumes. By shifting the port location for one or more of the surgical instruments, the amount of overlap may increase or decrease. The processor uses the overlap to optimize. The port locations are optimized with the goal of the workspace overlapping with all of the internal volumes. Some instruments may be optimized separately as different instruments are used for different internal volumes or anatomy interaction. The port locations may be optimized where a threshold amount of overlap occurs or where the maximum amount of overlap occurs. Any sampling of the port locations may be used, such as using a guideline or default port location and defining N number of other port locations relative to the initial location (e.g., 3x3 grid of port locations).

In one embodiment, parts of the internal volumes outside of the operating region or workspace of the surgical robotic system at the current port locations are identified. The identification may be for each of the robotic arms 1139 or as an overall combination for the arms 1139. The arm 1139 and corresponding instrument 1159 resulting in the greatest or largest part of the internal volumes outside the workspace is identified. The port location for that arm 1139 is moved to another possible port location. The process continues until optimal overlap for all arms 1139 occurs. Other search techniques may be used, such as testing each possible combination of port locations and selecting the combination with a greatest overlap.

In act 430, the processor 340 generates an indication of the port placement. The indication is displayed. In one embodiment, the indication is a graphic overlay on an image, such as showing a camera image of the patient on an operating table where graphics highlight the port locations for the different surgical instruments 1150. In another embodiment, the display is on the patient. A laser or other light pointer projects a graphic (e.g., red circle) onto the patient, where the graphic indicates the port location.

The indication of the port location or locations is displayed as part of the pre-operative plan. The port locations, determined based on the optimized overlap and/or internal volumes from the machine-learned model, are displayed for use in controlling or planning to control the surgical robotic system.

In one embodiment, the surgical robotic system has a coordinate system aligned with the patient. The indication is displayed for confirmation of acceptance by the surgeon. The surgical robotic system then moves the surgical instruments to the port locations automatically.

The indication may be output to memory, a display, robotic system controller, medical record, and/or a report. The processor 340 generates the indication as text, a light, a flag, graphic, or image. For example, the user interface displays the indication. The display is configured by loading an image with the indication into a display buffer or plane of the display device.

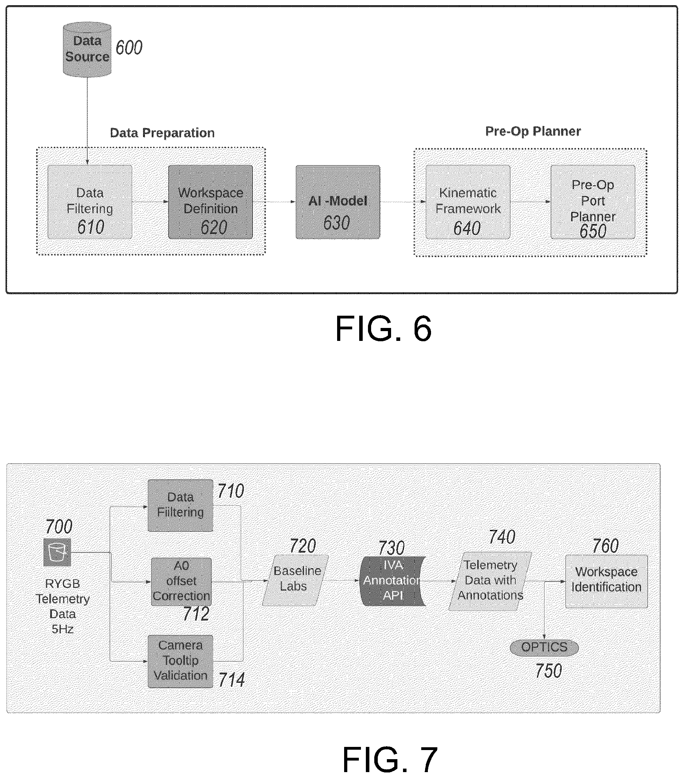

Figures 6-20 teach various embodiments of the system of Figure 3 and/or the method of Figure 4. For training data, various labs are obtained. The labs are previous surgical procedures using the surgical robotic system for a given surgery (e.g., RYGB). These embodiments use the artificial intelligence to output internal regions or tissue-interaction workspaces (anatomical locations) for the surgery, which output is then used in an optimization to determine the port location(s).

A pre-surgical assistance system framework for optimal port placement is shown in Figure 6. The framework can be divided into three major parts - data preparation, data modeling, and pre-op planner. A data source 600 is a database of source data, such as records of previous surgeries, such as on cadavers. The data preparation to train the artificial intelligence includes filtering 610 and workspace definition 620. The artificial intelligence 630 represents training the model using the prepared data. The resulting trained model is used in the pre-op planner. The pre-op planner includes the kinematic framework 640 to define the workspace or operating region for surgical instruments given port locations. The port planner 650 uses the output internal tissue regions from the trained artificial intelligence 630 and the operating regions from the kinematic framework 640 to optimize the port locations.

Figure 7 shows an embodiment of the data preparation. At a high-level, this part of the framework involves: 1) pre-processing unstructured data (telemetry 700 and surgical video data) from the robotics platform to filter 710 out noise, 2) transform workspaces across different patient types into a common frame of reference for the purpose of quantifying inter-patient anatomical variations (e.g., offset correction 712 to normalize across labs or previous surgeries), 3) validating 714 the surgical instruments 1159 involved from camera images, 4) formalize workspace definition based on Clinical Engineering (CE) procedure guidelines or baseline labs 720, 5) manually annotate 730 workspaces using C-SATS video annotation tool, IVA, or another tool, and 6) identify 760 the workspace from the telemetry data with annotation 740 with confirmation using optics 750.

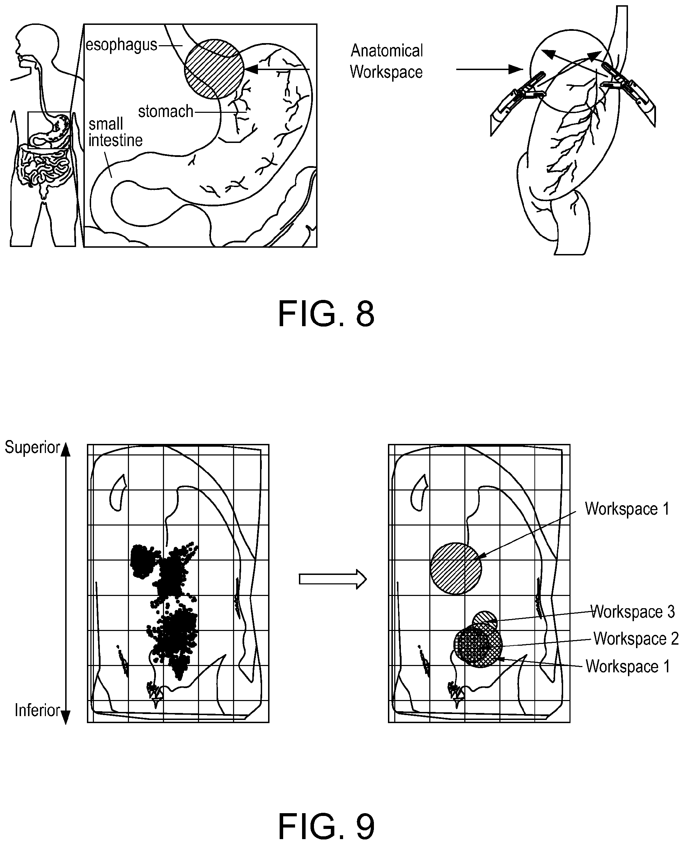

In this framework, anatomical workspace is the volume in which multiple robotic arms are to operate to successfully complete a procedure step. Figure 8 shows an example spherical volume as the anatomical workspace. Anatomical workspace is represented by the best fitting bounding sphere or other parameterization (e.g., mesh) that captures most of the tooltip points from the instrument arms for interaction with tissue. Figure 9 shows four different workspaces (right side) based on point locations of interaction from previous operations (left side). In summary, the data preparation takes raw telemetry data from the robotics platform and outputs centroids and workspace volumes for all previous labs or sample surgeries in a common frame of reference. This data preparation creates the training data for machine learning.

The robotic platform generates two unstructured data streams: 1) telemetry data 700 and 2) surgical video data. Both data streams are time-synced, structured, and filtered 710 before any further data processing. Manual methods may be used to time-sync telemetry and surgical video data. Combination of time-synced telemetry data and surgical procedure step data may be used to identify 3-dimensional locations of various anatomical sites. Lastly, a combination of filters separates separate surgical noise from valid data points.

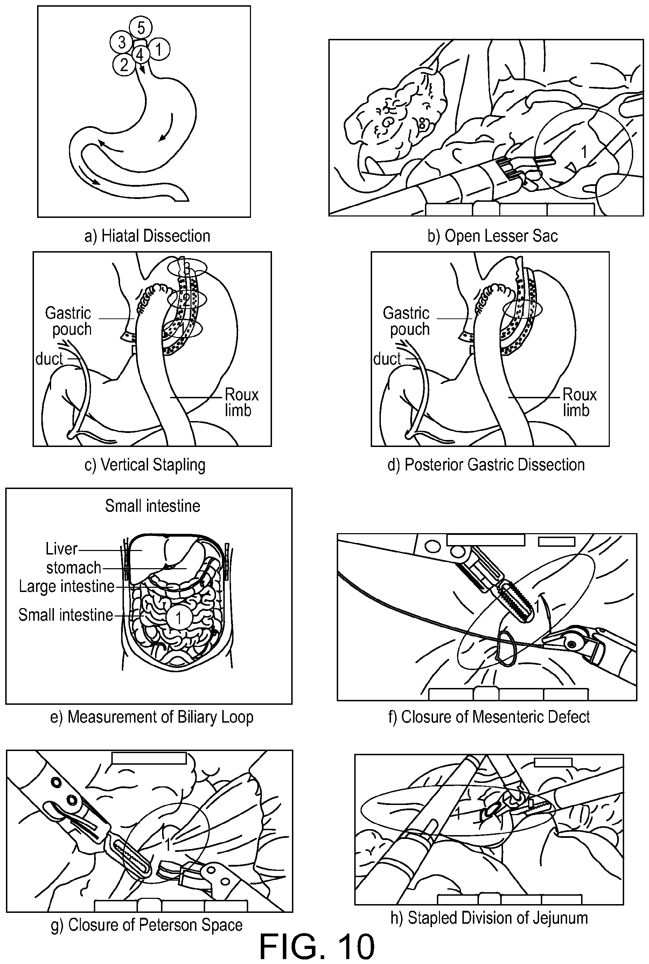

Clinical Engineering (CE) procedure guides are studied to identify the methods for segmenting specific surgical targets. As per the guidelines, the overall surgery is segmented into various procedure steps that represent specific tasks. Figure 10 shows RYGB separated into eight tasks. The data in each procedure step could potentially include regions from multiple target anatomies. Each procedure step could include data from multiple surgical tasks. Data in procedure steps may have inconsistencies and be mislabeled as procedure steps are progressed manually throughout the procedure by the operating surgeon. To overcome the limitations in the procedure step data, the telemetry data is segmented or separated differently to identify anatomical workspaces that represent unique anatomical regions. The anatomical workspace is represented by the best fitting bounding sphere that captures most of the tooltip points from the instrument arms, and its radius is computed as the knee of the tooltip point distance from the centroid.

A three-fold criterion is used for selecting the final workspaces: first, a large percentage of the labs should have data for that workspace; second, the workspace should represent a unique anatomical region; and third, the inter lab variation in the centroid location and radius should be within reasonable bounds. This three-fold criterion in applied to RYGB dataset to identify the workspaces. In one embodiment, there are a total of 31 workspaces in RYGB. In another embodiment, there are 15 workspaces, such as reducing the 31 workspaces to 15 for workspaces that failed to meet one or more of the requirements. For example, Preparation of Jejunostomy Stapling is only seen in 0.04% of 25 labs to be used as training samples, and hence that workspace wasn't considered in the final selection. Similarly, as shown in Figure 10, both horizontal stapling and Open Lesser Sac represent the same anatomical region, so horizontal stapling is not separately considered in the final selection. Similarly, Division of Omentum was excluded due to high inter lab variability. Table 1 summarizes fifteen unique anatomical workspaces identified for RYGB (See Figure 10 for the schematic).

**Table 1: List of Unique Anatomical Workspaces for RYGB**

| **Segment Name** | **Number of Workspaces** |
|---|---|
| Mobilization of Stomach - Hiatal Dissection | 5 |
| Mobilization of Stomach - Opening of Lesser Sac | 1 |
| Gastric Pouch Creation - Vertical Stapling | 3 |
| Gastric Pouch Creation - Posterior Gastric Dissection | 1 |
| Measurement of Bowel - Measurement of Biliary Loop | 1 |
| Closure of Mesentery - Closure of Mesenteric Defect | 1 |
| Closure of Mesentery - Closure of Peterson Space | 1 |
| Jejunal Division - Stapled Division of Jejunum | 1 |

For Workspace annotation 730, using the workspace definitions, the surgical videos are manually annotated using a C-SATS video annotation tool, IVA. These annotations are then turned into time points and used to segment out telemetry data into specific surgical targets in the 3D space. In other embodiments, a machine-learned model, such as an unsupervised segmentation model may be used to autonomously segment out the anatomical workspace volumes.

For a common frame of reference, to quantify variations in anatomical workspaces across different patient types, the segmented tooltip data from different labs are transformed into a common frame of reference (CFR) so that common functional spaces can be explored. A CFR that is independent of patient positioning and other variable constraints is selected. The external frame of reference on the patient surface may be used. In another embodiment, the CFR is the most superior operating region in the procedure because more interior regions of anatomy shift within the body during the procedure and has a consistent geometric relationship with other anatomical targets. In the case of RYGB, the CFR is the Angle of His (AoH) anatomical region. Other regions may be used. The frame of reference like AoH can be input using external landmark locations such as rib locations and/or distance between Xiphoid and Umbilics using a machine-learned model. In this way, the training and deployment of the machine-learned model is not dependent on the internal frame of reference.

Figure 11 describes a methodology for transforming tooltip telemetry data from various labs into a common frame of reference. Figure 11 shows an example where the angle of an anatomical region is annotated 1170 for all labs, the anatomical volume is segmented 1172 across the different labs, the largest anatomic volume is assessed 1174 to capture a conservative frame of reference, and transforms are computed 1176 for each lab with reference to the largest volume (e.g., calculate the delta offset of AoH for every lab by subtracting its own AoH from that of AoH of the reference lab and add the delta offset to all the tooltip points in the current lab).

In the current framework, the IVA videos are annotated for various workspaces so that the video-synced telemetry data can be used in various machine learning models. This labeling process requires the user to watch entire procedures and mark time segments at which specific workspaces occur. Not only is this a significant time investment but also is subject to human error and variation, especially as more annotators are used for labeling. Human annotations may be replaced using unsupervised segmentation technique to autonomously identify the anatomical workspaces.

A clustering algorithm, such as OPTICS, is used with heuristics to cluster point-cloud data for autonomously segmenting telemetry with varying densities and as unlabeled. In principle, OPTICS algorithm linearly orders the telemetry data such that spatially closest points become neighbors after ordering. Furthermore, a special distance is stored for each point that represents the density that must be accepted for a cluster so that both points belong to the same cluster.

The raw telemetry data is first passed through the filtering process. Next, telemetry and associated timestamp for each point is passed as an input for OPTICS. Specifically, each input consists of (x, y, z) coordinates of the left and the right arm, and the scaled timestamp. Time was scaled such that each second from the start of the procedure is equivalent to a change in distance by 1 mm. In addition to these inputs, OPTICS hyperparameters are tuned based on the inherent density properties of the dataset. These hyperparameters include: 1) minimum samples: the minimum samples in a neighborhood to be considered a core point; 2) minimum clusters: minimum points in a cluster to be considered; and 3) Xi: the steepness cutoff for the OPTICS reachability plot. A combination of grid and bisection search are employed to autotune these hyperparameters. Each run of hyperparameters is evaluated against the workspace annotations for that lab. That is, the spherical workspace from the annotations is compared to its most closely matching OPTICS cluster using IoU. The median IoU across all the workspaces is then taken as the final score to evaluate that run's hyperparameters.

The current workspace representation could have a lot of variation from lab to lab and may not be accurate representation in certain labs. Common sources of this variation include surgeon technique, random movements in the procedure, and human error in annotations. This may negatively impact the predictive power of the machine learning models. While standardizing labeling protocol and/or acquiring more lab data on workspaces could potentially reduce this variation, both approaches require significant resources and time. To address this issue, a convolutional neural network (CNN)-based feature identification pipeline capable of identifying unique visual features and/or signatures of a given workspace is used. In principle, filtering workspaces down to these unique signatures and/or features (i.e., the corrected workspace) produces more accurate workspace predictions.

A pipeline identifies the unique, visual signatures of a workspace, and a variation on region-based convolutional neural networks (R-CNN) is employed for this purpose. Figure 12 shows an embodiment. Firstly, the telemetry data 1200 for the anatomical workspaces is annotated. (x, y, z) coordinates of the annotated telemetry data is extracted for each workspace. As mentioned earlier, this annotation or labeling step is one of the sources of variation in the original workspaces.

To use 3D point-clouds in a 2D CNN, each point-cloud for each workspace is broken down into a 3x 2D projections 1202, one for each plane (i.e., x-y, y-z, and x-z). Data augmentation is added by varying the magnification of these projection from 4x to 0.5x at the center of the image. These projections are used an input into the first set of CNNs 1206. These images in turn are used to train 1204 multiple CNNs (the number of CNNs varies and is referred to as "training iterations" in Figure 12).

After creating and labeling these inputs, each CNN is trained 1204 on a random subset of labs. To prevent the model from identifying erroneous features induced due to human error in labeling, the model is trained up to an epoch 1210 at which it is still underfit and thus ensuring that the model captures more common and/or generic features of that workspace. The CNN 1206 is trained 1208 up to an epoch at which training converges. The epoch at the knee of the training loss curve.

Next, the filters for all the CNNs from a single convolution layer are clustered 1212 using K-Means clustering into several clusters that matches the number of filters in that layer. Then the "best" filter is determined from each cluster (currently "best" is defined as the cluster centroid or average filter of the cluster). These "best" filters are then placed 1214 into an untrained CNN with the same architecture as the previous CNNs. This is repeated for each convolution layer in the base CNN architecture to create a final CNN 1216 with these "best" filters. By clustering 1212 the filters, each cluster should represent a unique feature or texture of the image that was picked up by multiple CNNs trained on different subsets of labs. This final CNN 1216 is then used to create workspaces.

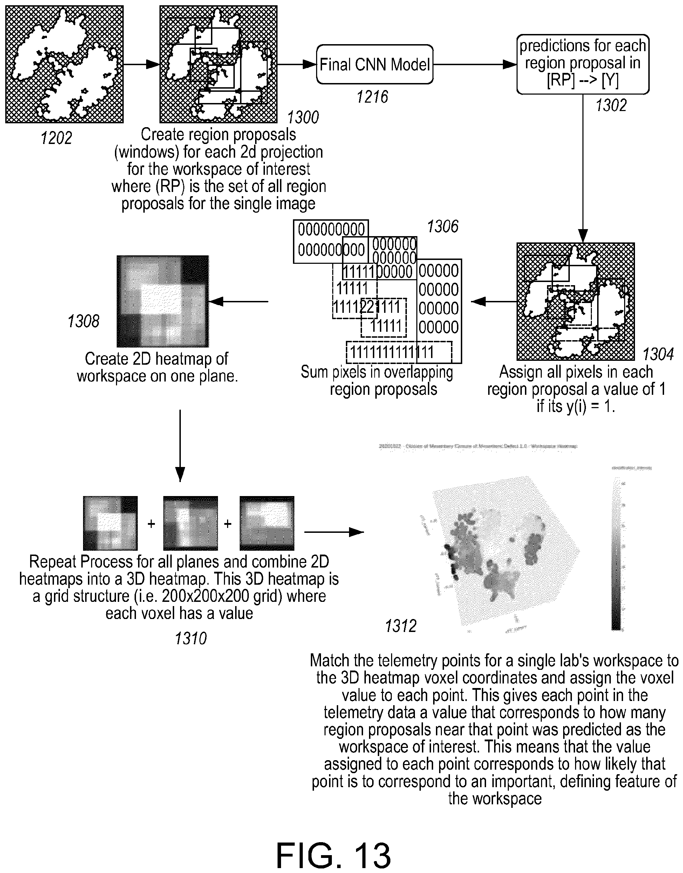

Figure 13 illustrates one embodiment of use of the final CNN 1216 to create workspaces. A modified R-CNN approach is used to create the "corrected" workspaces. Firstly, region proposals 1300 of the 2D projections 1202 at 1x magnification are created using a selective search algorithm. Region proposals 1300 are random windows or crops of the image 1202 that still capture relevant content (i.e., region proposals will not be all white or all black crops in this instance). These region proposals 1300 are then inputted into the final CNN model 1216 created earlier, outputting a prediction 1302 of 1 or 0, indicating if the region proposal 1300 was identified as the workspace of interest that the models were trained on. Starting from a matrix 1306 of all zeros, a value of one is added 1304 to the pixel values of positive region proposals. This creates heatmaps 1308 of where the 2d projection 1202 is most positively predicted 1302 as the workspace of interest and thus heatmap intensity would then correspond to strength of an identifiable feature of that workspace. These 2d projections 1202 are then back projected and added together to create a 3D heatmap 1310. The original points in the 3d point cloud are then matched 1312 with a voxel in this 3D heatmap and assigned that voxel's heatmap intensity. Once a threshold is applied to this heatmap value, all points above this threshold are identified as the "corrected workspace."

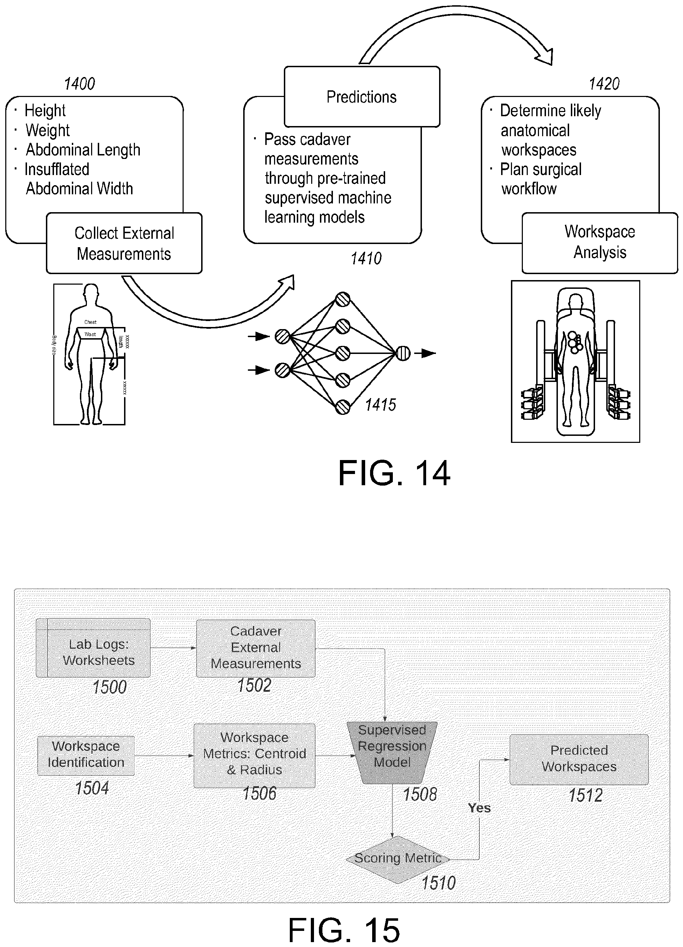

The prepared data is used to machine train a model. The workspaces are the ground truth internal volumes or regions of the patient for tissue interaction during surgery. The output from data preparation step (i.e., centroid and radius for all anatomical workspace volumes for all labs) and input data (e.g., external anatomical measurements for corresponding labs and/or surgeon profile extracted from lab logs) are fed into the data modeling. Figure 14 shows an example embodiment where inputs 1400 (e.g., external measurements in this example) are applied 1410 to machine-learned models 1415, which outputs anatomical workspaces or internal regions 1420. The main objective is to create one or more machine learning models that best explain the functional relationship between the input (e.g., external anatomical measurements) and geometrical properties (centroid and radius) of anatomical workspace volumes (i.e., the output). These models learn to predict the geometrical properties (centroid and radius) of various anatomical workspaces taking the external anatomical measurements for that specific lab as an input.

In some embodiments, classical regression or neural network models are used. Both neural network and classical regression models perform well for RYGB labs. Classical regression model performance may be better than that of neural network model. Geometrical properties (centroid and radius) of workspaces are predicted using machine-learned models. Depending on the model and procedure, characteristics one of the other approach (classical regression vs deep learning of a neural network) may be sufficient in identifying the anatomical volumes.

In one embodiment, regression is used to train the model, resulting in a machine-learned model or regressor. Figure 15 shows an example. Different models 1508 may be trained for different anatomical internal regions. The workspace 1504 of interest is identified, and the ground truth workspace metrics 1506 are obtained. The lab logs 1500 are used to extract the sample input measurements 1502, resulting in the training data for the supervised regression model 1508.

A varied list of classical regression models 1508 (e.g., linear, Ridge, SVR, decision tree, Lasso, ...) is searched through to find the best independent model 1508 that minimizes the error for each of the predicted variables (x, y, z, r) for a given workspace 1512 (here (x, y, z) and 'r' respectively represent the centroid and radius of the workspace). These are further analyzed to check for any cross-correlations between the predicted variables. Scenarios in which such correlations exist, dependent models 1508 were created using the best independent models 1508 as starting points.

For independent model generation, various linear and non-linear models 1508 are trained using cross-validation on the given cadaver measurements 1502 to predict the spatial (x, y, z) and volumetric (r) components of the annotated point cloud of a given segment 1512. These models 1508 are trained independently on each of the output variables 1506 using a variety of train/test splits (60/40, 70/30, 80/20, 90/10). Models 1508 are then scored 1510 separately for each output variable using the weighted mean scoring method, with error as the metric. The model with the highest score is kept as the best model for that segment and that output variable.

For dependent model generation, the Spearman rank correlation technique is employed to check for the existence of cross-correlations between predicted variables. For the cases in which such correlations exist, a regressor chain is created by feeding best predicted values for one output variable as an input for predicting the other dependent output variable. This regressor chain is then reversed to test both dependency directions.

For final model scoring, all independent and dependent model combinations are cataloged and scored using the weighted mean volumetric IOU technique. Error in centroid prediction and radius are used to compute the volumetric overlap between the predicted and original workspaces. Weighted average of these metrics is computed for all the labs in the test dataset to generate the final model score. The model combination with the highest score is kept as the best model for that segment and that output variable.

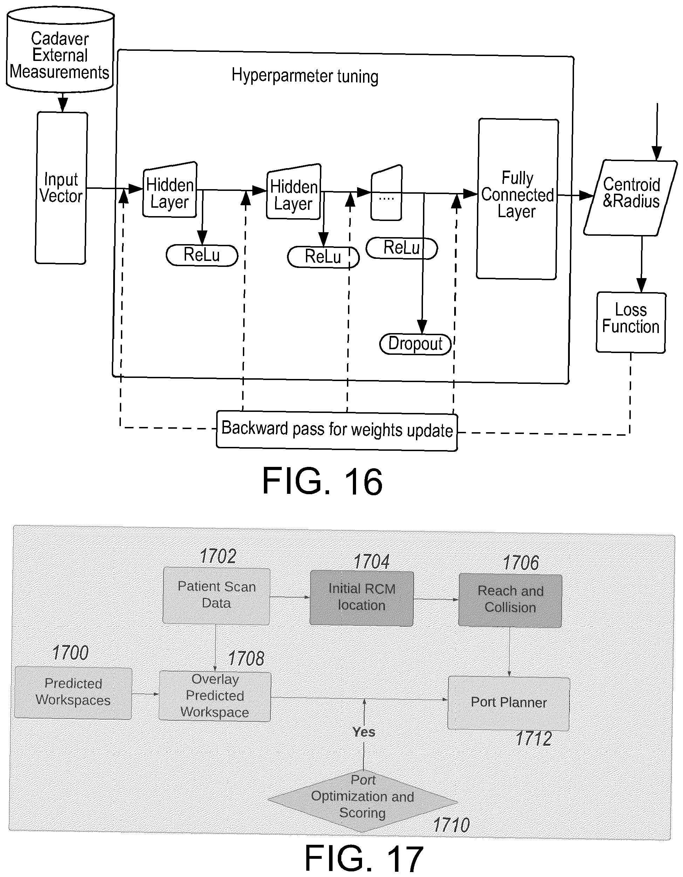

In other embodiments, one or more neural network models are used. For example, a multi-layer perceptron for each distinct workspace is trained using patient external measurements as the input vector and estimated internal geometries vector as output vector. Figure 16 shows an example neural network. Unique models are identified for each workspace defined based on the point cloud representation.

Any neural network architecture may be used. Figure 16 shows one example. The neural network architecture is a multi-layer perceptron (MLP) with ReLu activation and dropout layer for each data cohort. Various hidden layers with ReLU activation operate in sequence to feed to a dropout layer, followed by a fully connected layer, which outputs. For training, the input vector is used to estimate the output centroid and radius. A loss from the ground truth is used to backward pass for updating the weights. The model hyperparameters are tuned using a Bayesian optimization search to capture training parameters including learning rate, optimizer, hidden layer size and dropout probability.

For training and validation, the MLP model is trained using Monte Carlo Cross Validation (MCCV). In the MCCV, the dataset was randomly split into learning and test sets. For each split, the patient cohort is randomly split into training and test at different split ratios (e.g., split ratios of [0.1, 0.2, 0.3, 0.4]). Within each training set, 20% of the data is set on hold for model validation to prevent overfitting. Model performance for each train-test combination is evaluated using the test dataset. The process is repeated multiple times for a given split ratio to ensure all patient permutations are captured.

The anatomical modeling framework is surgery and/or procedure agnostic. For example, the same modeling may be used for the bariatric procedure Cholecystectomy. Cholecystectomy is a bariatric procedure that removes the gallbladder. At the beginning of the procedure the gallbladder is hidden from view by the liver, as such the gallbladder fundus is retracted above the liver to expose the omental adhesions that surround the base of the gallbladder. After these adhesions are removed, calot's triangle can be exposed. Once Calot's triangle is exposed, a critical view of safety is achieved by skeletonizing the cystic artery and duct and removing the bottom 1/3 of the gallbladder from the hepatic plate. Only after the critical view of safety is achieved are the cystic artery and duct clamped and cut. The gallbladder can then be removed from the hepatic plate, the hepatic plate can be cauterized, and the gallbladder can be removed from the body. Thus, the surgery includes various internal regions as anatomical workspaces, so the modeling may perform similarly as RYGB for this surgery.

The workspace representation may have a lot of variation from lab to lab and may not properly represent the workspace in certain labs, which may have an adverse effect on machine-learned-model predictability. Autonomously identifying unique signature and/or features of different workspaces may enhance the model accuracy.

For optimization of the port location, the processor implements a pre-op planner using the machine-learned model or models. The robot's coverage space is based on port location and robot arm 1139 and surgical instrument 1159 design. The coverage or operating space is the total volume of space that is reachable without collisions by the surgical instrument 1159 for a given port location. A total volume as a sum of multiple instruments 1159 on different robotic arms (i.e., through different ports) may be used. The overarching goal is to determine the impact of moving port locations on the coverage of the predicted workspaces (anatomical internal regions) to potentially suggest more optimal port locations for maximal coverage (i.e., overlap of the operating space with the internal volumes). Maximal coverage helps ensure that critical anatomical structures are reached without collisions during the procedure for a new patient.

In one embodiment, the analysis includes loading lab data used to train the machine learning models, and utilizing the inverse kinematics of the robot to map out the total volume reachable by all the active instrument arms 1139 without collisions. For validation, golden datasets may not be available. The ground truth collected in labs is used. How a lab went is known. This is run through the models to estimate what the predictions might be. Then, the outcome is matched via prediction and actual outcome in labs to validate the closeness to reality of the outputs produced by the machine-learned model The total volume is the workable area and is a conservative estimate of total robot workspace. This total volume in turn is used to evaluate coverage amounts and is fed into port movement heuristics. The surgical assistance system framework predicts the geometrical properties of anatomical workspaces using the external measurements. These predicted workspaces in turn are used to determine the pre-op optimal port placement locations.

Figure 17 shows one embodiment. The machine-learned models predict geometric properties (centroid and radius - internal regions or anatomical workspaces 1700) of various anatomical workspaces. The anatomical workspaces are overlaid 1708 on patient scan data 1702, such as pre-operative scan. Based on a guideline or other default, an initial port location (RCM) 1704 is used to determine the reach and collision avoidance 1706 using inverse kinematics. The initial location may be a best guess port input by the physician. Port optimization 1710 uses an overlap score for port planner 1712 to optimize the port location.

The first pass of this analysis involves evaluating the kinematic feasibility for a test lab under consideration. This involves quantifying the minimum amount by which the current port(s) location(s) need to be moved so that the overall robot coverage score is greater than or equal to a pre-specified threshold value (e.g., 95%). The movement of any of the active instrument ports may be limited, such as limiting to be within 10mm from the original port location. Moving any port location affects the coverage volumes of both the robot and the ML workspaces.

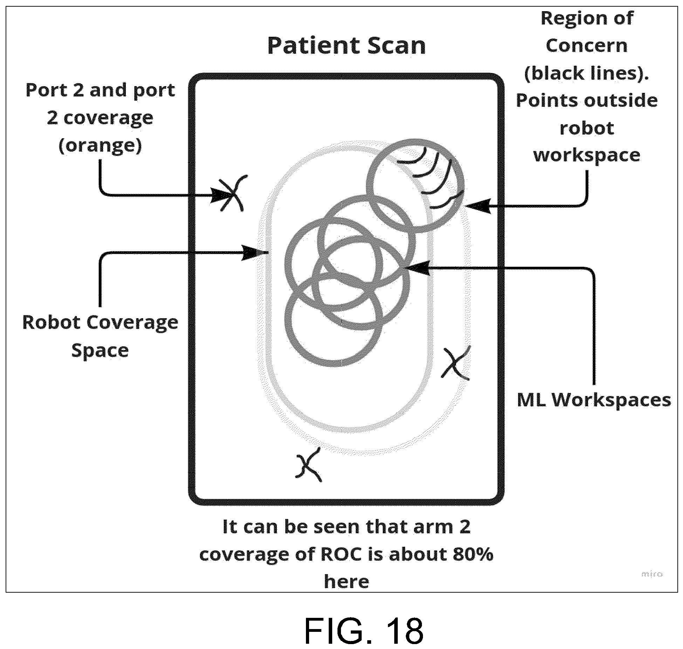

Figure 18 illustrates the optimization involved in evaluating the kinematic feasibility. As it can be seen, this process involves first identifying all regions of concerns (ROC) i.e., identify the regions in ML workspaces that are unreachable by the robotic arms. Next, the active arm 1139 that has minimum coverage in the region of concern is identified. The port location for that arm 1139 is the primary candidate for the port movement. Next, a greedy search is employed where the candidate's current port location is moved to a new port location within 3x3 grid. The coverages and the associated ROC scores are re-calculated. This process terminates when a maximum coverage score is greater than or equal to a pre-specified threshold or a maximum number of iterations is reached (whichever happens first).

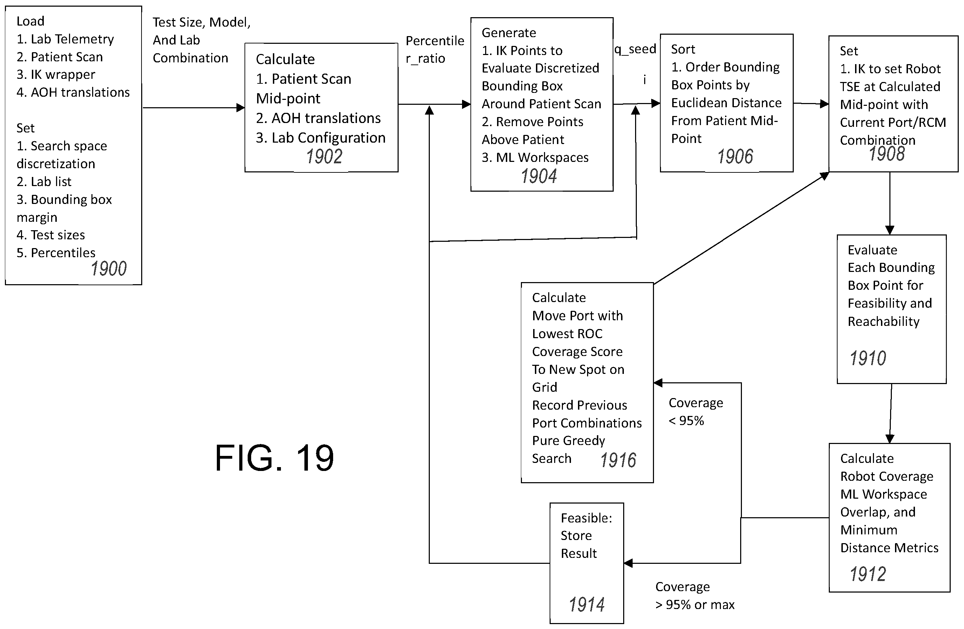

Figure 19 is one embodiment of a workspace analysis flowchart. First, patient scans, RYGB lab data and the inverse kinematics (IK) production software wrapper are loaded 1900. At 1902, the midpoint of the patient scan is assigned as the initial search point for the IK wrapper. At 1902, AOH transformations are applied to the machine-learned (ML) anatomical workspace data to overlap precisely with the patient scan and the original lab data. At 1904, a discretized bounding box is created around the patient scan and each point within this box is evaluated for feasibility, reachability and collisions using the inverse kinematics solver. The output of this analysis is a concave hull that represents the robot workspace. At 1906, the points are ordered based on the Euclidean distance from the patient midpoint to enable a breadth-first search to ensure that the inverse kinematics does not evaluate points that are too far apart from each other. In addition, this also ensures that the IK wrapper seed configuration always uses the closest feasible solution found from the preceding points that have already been evaluated. At 1908, the MATLAB generalized IK is used to bring the robot to this initial starting point (patient midpoint) to start the search. At 1910, each bounding box is evaluated for feasibility and/or reachability. The solution for each independent arm is simultaneously stored. Once the robot space is calculated at 1912, if coverage is less than 95% or max iterations is not yet reached, the port with the least ROC score is moved at 1916 to a new position on the 3x3 grid and the search re-starts.

In one embodiment, a maximum port combination iteration of 20 is used. The points within the bounding box are discretized to 2.75cm for the neural network and 3.5cm for the classical regression model for speed. An extra margin of 10cm is used to expand the bounding box to account for poorly predicted workspaces.

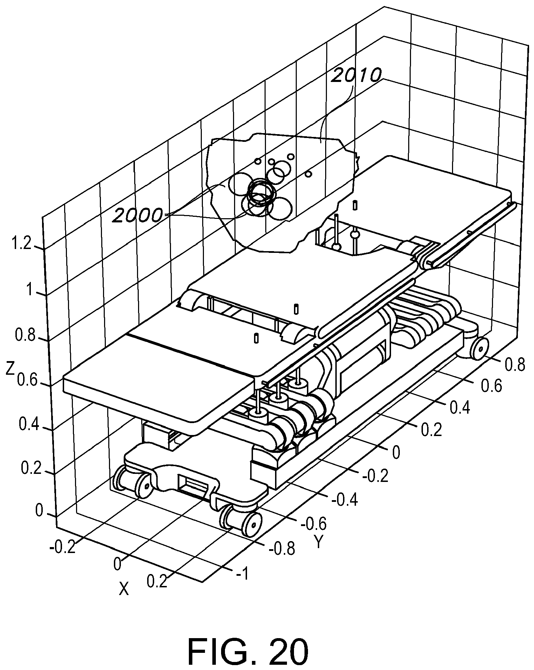

Figure 20 illustrates internal volumes or anatomical workspaces 2000 predicted by the machine-learned model. These internal regions 2000 are shown as darker shading. The surgical robotic system workspace or operating region 2010 is shown with lighter shading and completely covers the internal regions 2000.

The above description of illustrated embodiments of the invention, including what is described below in the Abstract, is not intended to be exhaustive or to limit the invention to the precise forms disclosed. While specific embodiments of, and examples for, the invention are described herein for illustrative purposes, various modifications are possible within the scope of the invention, as those skilled in the relevant art will recognize. These modifications can be made to the invention in light of the above detailed description. The terms used in the following claims should not be construed to limit the invention to the specific embodiments disclosed in the specification. Rather, the scope of the invention is to be determined entirely by the following claims, which are to be construed in accordance with established doctrines of claim interpretation.

## Claims

1. A method for port placement recommendation for a surgical robotic system, the method comprising:
inputting a plurality of external patient measurements of a patient to a machine-learned model;
in response to the inputting, outputting by the machine-learned model one or more internal volumes of the patient;
optimizing overlap of an operating region of the surgical robotic system with the one or more internal volumes based on the port placement; and
displaying an indication of the port placement from the optimized overlap.

2. The method of claim 1 wherein inputting comprises inputting the external patient measurements as height, weight, gender, body mass index, abdominal width, and pelvic location.

3. The method of claim 1 wherein the machine-learned model comprises a regression trained model, and wherein outputting comprises outputting by the regression trained model in response to the inputting of the external patient measurements.

4. The method of claim 1 wherein the machine-learned model comprises a multi-layer perceptron neural network, and wherein outputting comprises outputting by the multi-layer perceptron neural network in response to the inputting of the external patient measurements.

5. The method of claim 1 wherein outputting the one or more internal volumes comprises outputting a centroid and radius for each of the one or more internal volumes.

6. The method of claim 1 wherein optimizing comprises identifying the operating region of the surgical robotic system with inverse kinematics.

7. The method of claim 1 wherein optimizing comprises identifying a part of the one or more internal volumes outside of the operating region for a port location for each of a plurality of arm of the surgical robotic system, identifying the arm of the surgical robotic system associated with a larger part, and changing the port location.

8. The method of claim 1 wherein displaying comprises displaying the indication as part of a pre-operative plan.

9. The method of claim 1 wherein inputting further comprises inputting a profile of a surgeon to the machine-learned model, and wherein outputting comprises outputting the one or more internal volumes in response to the inputting of the profile and the external patient measurements, the one or more internal volumes accounting for the profile of the surgeon.

10. A method for port placement recommendation for a surgical robotic system, the method comprising:
inputting a profile of a surgeon to a machine-learned model;
in response to the inputting, outputting by the machine-learned model one or more internal volumes of a patient, the one or more internal volumes accounting for the profile of the surgeon; and
displaying an indication of port placement based on the one or more internal volumes.

11. The method of claim 10 wherein outputting comprises outputting the internal volumes as regions for surgical interaction based on a characteristic of the surgeon reflected in the profile.

12. The method of claim 11 wherein the characteristic is handedness of the surgeon or surgical trajectory used by the surgeon, and wherein outputting comprises outputting the internal volumes based on the handedness or the surgical trajectory.

13. The method of claim 11 wherein inputting further comprises inputting external measurements of the patient to the machine-learned model, and wherein outputting comprises outputting the one or more internal volumes based on the external measurements of the patient.

14. The method of claim 13 further comprising optimizing overlap of an operating region of the surgical robotic system with the one or more internal volumes based on the port placement, and
wherein displaying comprises displaying the indication of the port placement from the optimized overlap.

15. A surgical robotic system for port placement recommendation, the surgical robotic system comprising:
a robotic arm configured to hold and operate a surgical tool; and
a processor configured to determine a location of a port for the surgical tool to enter a patient, the location determined from an output of artificial intelligence generated in response to input of patient measurements and surgeon information.

16. The surgical robotic system of claim 15 wherein the output of the artificial intelligence is one or more internal regions of the patient, the one or more internal regions being regions for interaction of the surgical tool with the patient, and wherein the processor is configured to optimize the location of the port based on overlap of the one or more internal regions and an operating region accessible by the surgical tool using the robotic arm, the operating region based on inverse kinematics.

17. The surgical robotic system of claim 16 wherein the surgeon information comprises a handedness of a surgeon controlling the robotic arm, the one or more internal regions being positioned based on the handedness.

18. The surgical robotic system of claim 16 wherein the surgeon information comprises a trajectory of a surgeon controlling the robotic arm, the trajectory being a sequence or route to be used by the surgeon during operation on the patient with the surgical tool, the one or more internal regions being positioned based on the trajectory.

19. The surgical robotic system of claim 15 wherein the patient measurements comprise external measurements of the patient.

20. The surgical robotic system of claim 15 wherein the artificial intelligence comprises a machine-learned regression model or a machine-learned neural network.
